# EUROPÄISCHE PATENTSCHRIFT

(11) **EP 3 496 780 B1**
(45) Veröffentlichungstag und Bekanntmachung des Hinweises auf die Patenterteilung: **04.08.2021**
(21) Anmeldenummer: 17745965.8
(22) Anmeldetag: 14.06.2017
(51) Int. Cl.: A61M 1/16, A61M 1/32, A61M 1/36, A61F 7/00

(54) **OXYGENATOR MIT EINER GEHÄUSEWANDUNG**
OXYGENATOR WITH A HOUSING WALL
OXYGÉNATEUR AVEC UNE PAROI DE BOÎTIER

(30) Priorität: 09.08.2016 DE 102016009599; 05.10.2016 DE 102016011946
(43) Veröffentlichungstag der Anmeldung: 19.06.2019
(73) Patentinhaber: Xenios AG, 74076 Heilbronn (DE)
(72) Erfinder: JÖCHLE, Knut, 74172 Neckarsulm (DE); KÖHLER, Sebastian, 74354 Besigheim (DE)
(74) Vertreter: Graf von Stosch, Andreas
(86) Internationale Anmeldenummer: PCT/DE2017/000162
(87) Internationale Veröffentlichungsnummer: WO 2018/028727

(56) Entgegenhaltungen:
- EP-A2- 2 143 453
- WO-A2-2004/105589
- DE-B- 1 096 554
- US-A- 3 112 746
- US-A- 3 211 148
- US-B2- 7 153 285

## Beschreibung

Die Erfindung betrifft einen Oxygenator mit einer Gehäusewandung, die einen Gehäuseraum mit einem Bluteinlass, einem Blutauslass, einem Gaseinlass und einem Gasauslass begrenzt, und einem Heizelement, um durch den Gehäuseraum fließendes Blut zu temperieren, wobei das Heizelement eine Strahlungsquelle und einen Receiver aufweist, der die Strahlung der Strahlungsquelle in Wärme umwandelt.

Oxygenatoren sind medizinische Gasaustauscher, welche hauptsächlich in mehrtägigen Herz-Lunge-Therapien oder bei Operationen eingesetzt werden. Ein weiterer Anwendungszweck ist bspw. die Dialyse. Diese Oxygenatoren bieten neben dem Gasaustausch häufig auch die Möglichkeit, durch einen Gehäuseraum des Oxygenators fließendes Blut zu temperieren. In der Regel wird das Blut im Oxygenator erwärmt, da die Bluttemperatur im extrakorporalen Kreislauf, also außerhalb des Patientenkörpers, im Laufe der Zeit abnimmt und der Patient unterkühlt wird. Neben dieser Erwärmung gibt es auch die Möglichkeit, bei Operationen am Herzen die Temperatur des Blutes zu kühlen, um die Körpertemperatur zu senken.

Um die Blut- und Körpertemperatur eines Patienten während einer Operation oder bei längeren Therapieeinsätzen mit Gasaustauschern zu regulieren, werden Heater-Cooler (HC-Geräte) eingesetzt. Ein Heater-Cooler (HC-Gerät) ist ein externes Gerät, welches über Schläuche mit einem Oxygenator verbunden ist. Im HC-Gerät wird Wasser durch Metallstreben geleitet und erhitzt oder gekühlt. Das Wasser wird anschließend zum Oxygenator geleitet und fließt durch Wärmetauschermatten aus Hohlfasern oder spezielle meist metallische Kanäle im Oxygenator, an denen das Blut vorbeigeleitet wird. Ein derartiger Oxygenator ist in der EP 765 683 B1 beschrieben.

Derartige Oxygenatoren sind praktisch im Einsatz. Die verwendeten Heater-Cooler-Geräte arbeiten jedoch mit einem Wasserbad, das während der Nutzung verschmutzt und die Luft der Umgebung kontaminieren kann. Die HC-Geräte sind wegen des Wasserbades und der Kühleinrichtung sehr schwer und unmobil. Sie müssen regelmäßig gereinigt werden, da sie in der Nähe des Oxygenators und somit im Krankenhaus z.B. in Operationssälen oder Intensivstationen eingesetzt werden.

Die US 3 112 746 A zeigt einen gattungsgemäßen Oxygenator. Ähnliche Oxygenatoren zeigen die DE 10 96 554 B, die EP 2 143 453 A2 und die US 3 211 148 A.

Der Erfindung liegt daher die Aufgabe zugrunde, einen Oxygenator weiterzuentwickeln. Diese Aufgabe wird mit einem gattungsgemäßen Oxygenator mit den Merkmalen des Patentanspruchs 1 gelöst.

Der Erfindung liegt die Erkenntnis zugrunde, dass eine Erwärmung im Oxygenator zwischen Bluteinlass und Blutauslass mittels Flüssigkeit führenden Röhren ein aufwändiges Zusatzgerät benötigt. Dadurch, dass das Heizelement eine Strahlungsquelle und einen Receiver aufweist, kann auf ein HC-Gerät verzichtet werden und es wird nur eine Spannungsquelle für die Strahlungsquelle benötigt.

Zwischen dem Bluteinlass und dem Blutauslass können auch andere Flüssigkeiten als Blut, wie beispielsweise Blutersatzlösungen wie Primingflüssigkeiten oder medizinische Lösungen wie Pufferlösungen mit Medikamentenzusatz fließen, im Oxygenator erwärmt werden. Wenn im Folgenden von Bluttemperatur gesprochen wird, so sind dabei auch derartige Flüssigkeiten gemeint.

Die Strahlungsquelle ermöglicht es, die Bluttemperatur in einem Bereich der Körpertemperatur und somit zwischen 36 und 38 °C zu halten. Dabei ist darauf zu achten, dass die Temperatur möglichst an keinem Ort der Vorrichtung 40 °C überschreitet. Da das Blut den Receiver abkühlt, liegt die Erwärmung des Receivers abhängig vom Blutfluss bei 35 °C bis 60 °C.

Die Erfindung sieht vor, dass die Temperaturregelung die Temperatur des Receivers an verschiedenen Orten individuell einstellt oder regelt. Dies ermöglicht es, auf der Grundlage der üblichen Strömungsgeschwindigkeiten im Oxygenator an unterschiedlichen Stellen mit unterschiedlicher Intensität zu heizen.

Dafür wird vorgesehen, dass der Receiver an unterschiedlichen Orten im Gehäuseraum eine unterschiedliche Heizleistung abgibt. Die Heizleistung kann je nach Blutfluss, Blutgeschwindigkeit, Gasfluss und Gasgeschwindigkeit variiert werden.

Eine Ausführungsvariante sieht vor, dass der Receiver mehrere an verschieden Orten des Oxygenators positionierbare Receiverteilelemente aufweist. Diese Receiverteilelemente können dann getrennt voneinander individuell angesteuert werden, um im Oxygenator eine bestimmte Heizintensitätsverteilung zu erzielen und diese gegebenenfalls auch während des Betriebs des Oxygenators zu ändern.

Eine erste Variante sieht vor, dass die Strahlungsquelle Infrarotlicht abgibt und der Receiver eine dunkle, vorzugweise mattschwarze Fläche, aufweist. Dabei kann die dunkle Fläche vom Blut selber gebildet werden, sodass die Infrarotlichtstrahlung beim Auftreffen auf das Blut Wärmeenergie an das Blut abgibt. Dadurch bildet die dunkle Fläche des Blutes den Receiver. Kumulativ oder alternativ kann der Receiver dunkle Flächen aufweisen, die zwischen der Strahlungsquelle und dem Blut angeordnet sind, um sich selbst zu erwärmen und anschließend das Blut. Die Receiverflächen können jedoch auch so angeordnet sein, dass das Infrarotlicht durch das Blut hindurch auf die Receiverfläche trifft und dabei einerseits das Blut direkt erwärmt und andererseits indirekt über die erwärmte Receiverfläche.

Als Infrarot wird der Spektralbereich zwischen 10⁻³ und 7,8 ^{∗}10⁻⁷ m (1 mm und 780 nm) bezeichnet. Dies entspricht einem Frequenzbereich von 3^{∗}10 ¹¹ Hz bis ca. 4^{∗}10¹⁴ Hz (300 GHz bis 4000 THz). Besonders geeignet ist nahes Infrarot NIR (IR-A mit Wellenlängen zwischen 0,78 und 1,4 µm und IR-B mit Wellenlängen zwischen 1,4 und 3,0 µm sowie mittleres Infrarot NIR (IR-C mit Wellenlängen zwischen 3 und 50 µm).

Die Eindringtiefe von Infrarotlicht ist dabei abhängig von der Wellenlänge der Strahlung und den Materialeigenschaften der Auftrittsoberflächen sowie gegebenenfalls der Filterung durch Gehäuseteile und einer zwischen der Strahlungsquelle und dem Receiver liegenden Blutströmung.

Da Oxygenatoren grundsätzlich aus transparentem Material, wie beispielsweise Polyurethan bestehen, können bekannte Oxygenatoren verwendet werden, in dem außerhalb des Gehäuses eine Strahlungsquelle mit Infrarotstrahlung angeordnet wird, die durch die äußere Gehäusewandung strahlt.

Derartige Receiver können in jegliche Bauform medizinischer Gasaustauscher integriert werden, um das Blut zu erwärmen. Dafür werden am Oxygenator oder im Oxygenator Oberflächen aus dunklem, besonders wärmestrahlungsabsorbierendem Material hergestellt oder Folien eingesetzt, die in dem Oxygenatorgehäuse angeordnet werden. Neben symmetrischen Anordnungen der Receiver im Gehäuse können auch strömungsadaptierte Anordnungen verwendet werden, die es ermöglichen, an Bereichen mit einem großen Volumenstrom besonders große Receiverflächen vorzusehen.

Vorteilhaft ist es, wenn der Oxygenator weiterhin einen transparenten Bereich aufweist, durch den ein Mediziner den Zustand des Blutes innerhalb des Oxygenators visuell überprüfen kann.

Um die Intensität des Infrarotstrahlers zu variieren, wird vorgeschlagen, dass der Infrarotstrahler mehrere Strahlungsquellen, wie beispielsweise mehrere dimmbare Lampen, Ionen oder Laser oder Filter aufweist, welche vorzugweise einzeln angesteuert werden können, um die Intensität der Erwärmung einzustellen. Daher ist es vorteilhaft, wenn einerseits die Wellenlänge der Strahlungsquelle einstellbar ist. Hierbei kann mit einem Algorithmus die Wellenlänge so variiert werden, dass unterschiedliche Eindringtiefen erzielt werden.

Andererseits ist es vorteilhaft, wenn die Absorptionsfähigkeit des Receivers geregelt veränderbar ist. Dies kann beispielsweise durch eine Farbänderung des Receivers oder eine Änderung der Position des Receivers relativ zur Strahlungsquelle erreicht werden. Hierzu kann beispielsweise ein Klappenmechanismus oder ein Richtschirm vorgesehen werden, um entweder das Infrarotlicht an verschiedene Stellen des Gasaustauschers zu lenken oder mit dem Receiver mehr oder weniger Infrarotlicht aufzunehmen. Dabei kann insbesondere die Bestrahlung mit der Strahlungsquelle so ausgerichtet werden, dass sie zeitabhängig unterschiedliche Bereiche des Oxygenators anstrahlt. Dadurch entsteht eine wandernde Bestrahlung. Bei einer automatischen Regelung werden diese Daten an die Konsole gemeldet, darauf aufbauend die Heizleistung steuert oder regelt.

Eine alternative Ausführungsform sieht vor, dass die Strahlungsquelle eine Induktionsspule und der Receiver ein induktionsfähiges Material aufweist. Ein induktionsfähiges Material ist beispielsweise Kupfer oder eine Eisenlegierung. Dadurch wird eine induktive Erwärmung möglich.

Die Strahlungsquelle kann beispielsweise eine große flache einlagige Spule aus Hochfrequenzlitzen sein, die ein hochfrequentes magnetisches Wechselfeld erzeugen. Diese Spule bildet mit Kondensatoren einen Schwimmkreis, der von einem oder mehreren Schalttransistoren in Resonanz versetzt wird. Die Leistungssteuerung kann durch unterschiedliche Schaltungskonzepte wie beispielsweise eine Transistorschaltung, eine Anregungsfrequenzsteuerung und eine pulsweiten Steuerung realisiert werden. Besonders vorteilhaft ist bei der induktiven Erwärmung, dass die Leistung sehr genau eingestellt wird.

Ähnlich wie bei einer Strahlungsquelle mit Infrarotlicht kann auch für eine Strahlungsquelle als Induktionsspule ein Richtschirm oder ein halbkugelförmiger Schutzschirm um die Strahlungsquelle vorgesehen sein. Dadurch kann magnetische Streu- oder Störstrahlung verhindert werden. Dadurch bleibt das magnetische Wechselfeld bzw. elektrische Feld räumlich begrenzt.

Um Überhitzungen zu vermeiden, kann die Strahlungsquelle einen Lüfter aufweisen. Diese Maßnahmen führen dazu, dass der Oxygenator und der Receiver sehr präzise auf die gewünschte Temperatur eingeregelt werden können.

Der Receiver kann in der äußeren Wandung des Oxygenators angeordnet sein, um im Gehäuseraum fließendes Blut zu erwärmen. Besonders vorteilhaft ist es jedoch, wenn der Receiver im Gehäuseraum angeordnet ist. Dies ermöglicht den Einsatz großer Receiverflächen und dadurch einen geringen Temperaturunterschied zwischen Receiverfläche und Blut. Dadurch wird eine Schädigung des Blutes vermieden.

Je nach Einsatzzweck kann der Receiver jedoch auch in der Gehäusewandung angeordnet sein. Dies ermöglicht einen einfachen Aufbau und insbesondere bei ebenen parallel gelegten Hohlfasermatten einen guten Wärmeübergang vom Receiver auf das Blut.

Zwischen den Gasbereichen und den Blutbereichen des Oxygenators sind in der Regel semipermeable Materialien wie insbesondere Membranen angeordnet. Diese Membranen können ebene Folien oder Hohlfasern sein.

Zum Halten von planen oder rohrförmigen Membranen in einem Oxygenator werden Vergussmaterialien bspw. aus Kunststoff eingesetzt. Daher ist es vorteilhaft, wenn der Oxygenator eine Vergussschicht zum Halten von Fluidleitungen aufweist und der Receiver in dieser Vergussschicht oder zumindest auch in dieser Vergussschicht angeordnet ist.

Bei der Erhitzung von Blut muss darauf geachtet werden, dass auch nur durch eine bereichsweise Überhitzung des Blutes keine Blutschädigungen auftreten. Daher wird vorgeschlagen, dass der Oxygenator mindestens einen Temperatursensor aufweist. Besonders vorteilhaft ist es, wenn im Oxygenator an verschiedenen Stellen Temperatursensoren vorgesehen werden, um sicherzustellen, dass in keinem Bereich zu hohe Temperaturen auftreten. Die Temperatursensoren sollten daher nach Möglichkeit zumindest auch in Bereichen angeordnet sein, in denen eine langsamere Blutflussgeschwindigkeit als in anderen Bereichen des Gehäuseraumes oder in denen eine langsamere Blutflussgeschwindigkeit als die mittlere Blutflussgeschwindigkeit im Gehäuseraum vorliegt und somit das Risiko einer Überhitzung bestehen kann.

Über die an der Strahlungsquelle angelegte Spannung kann die Temperatur des Receivers variiert werden und es ist daher sinnvoll, wenn der Oxygenator eine Temperaturregeleinrichtung aufweist.

Vorteilhaft ist es, wenn die Temperatur an einer oder mehreren Stellen in bestimmten Zeitintervallen gemessen wird. Die Frequenz kann durch einen Algorithmus vorgegeben sein. Dadurch können risikohafte Überhitzungen vermieden werden. Hierbei spricht man von Pulsweitenmodulation.

In vielen Fällen steht der Oxygenator in Verbindung mit einer Konsole, über die beispielsweise die Durchströmung des Oxygenators mit Gas oder Blut gesteuert werden kann. Eine derartige Konsole ist eine Steuerelektronik zum Steuern oder Regeln des Oxygenatoreinsatzes. Über eine derartige Konsole können somit auch die Strahlungsquelle und/oder der Receiver angesteuert werden und diese Ansteuerung kann in Abhängigkeit von anderen an der Konsole vorliegenden Daten oder Verfahrensparametern geregelt werden, wie Blut oder Gasstrom und der Temperatur im Oxygenator.

Ein Zusatzeffekt wird dadurch erzielt, dass der Receiver im Oxygenator zwischen Gaseinlas und Gasauslass angeordnet ist, um auch durch den Gehäuseraum fließendes Gas zu temperieren. Dadurch kann vor allem auch Kondensatbildung vermieden werden.

Eine einfache Ausführungsform eines Oxygenators sieht einen Oxygenator mit einer Gehäusewandung vor, die nur vier nach außen führende Fluiddurchgänge aufweist. Davon können zwei Fluiddurchgänge für den Gaseinlass und Gasauslass und zwei Fluiddurchgänge für den Bluteinlass und Blutauslass verwendet werden.

Ein Konnektor bezeichnet eine Anschlussmöglichkeit von Schläuchen am Oxygenator. In einem derartigen Konnektor kann ein Teil des Heizelements angeordnet sein.

Vorteilhaft ist es, wenn der Oxygenator eine Wärmeleiteinrichtung für eine Wärmeleitung zum Heizelement aufweist. Wenn das Heizelement beispielsweise als erwärmbares Metallteil ausgebildet ist, kann dieses zur Vergrößerung der Oberfläche oder um einen Kontakt zwischen Blut- und Metallteil zu verhindern mit einer Wärmeleiteinrichtung umgeben sein. Diese Wärmeleiteinrichtung leitet dann die Wärme vom Heizelement an eine Oberfläche, die mit dem Blut in Verbindung steht und vorzugweise größer ist als die Oberfläche des Heizelementes. Eine derartige Oberfläche kann die Oberfläche eines Netzes oder einer Folie sein.

Die Wärmeleiteinrichtung sollte insbesondere dazu dienen, eine Wärmeverteilung vom Heizelement im Gehäuseraum zu ermöglichen.

Eine vorteilhafte Ausführungsvariante sieht vor, dass der Oxygenator eine Isolationsschicht oder eine Vakuumschicht aufweist, um im Gehäuseraum fließendes Blut zu isolieren. Eine Isolationsschicht und eine Reflexionsschicht können auch so ausgebildet sein, dass sie geöffnet werden kann, um auch Wärme wieder leicht abzugeben, um den Oxygenator zu kühlen und somit eine Überhitzung zu vermeiden. Außerdem können die Schichten auch partiell angeordnet sein oder partiell anordenbar sein.

Um Wärmestrahlung von im Gehäuseraum fließendem Blut zum Blut zurück zu reflektieren und somit auch die Abgabe von Wärmestrahlung am Oxygenator zu minimieren, wird vorgeschlagen, dass der Oxygenator eine Reflektionsschicht aufweist. Eine derartige Reflektionsschicht kann beispielsweise eine Metallfolie sein oder eine polierte Oberfläche.

Damit die Blutströmung im Oxygenator beobachtet werden kann, ist es vorteilhaft, wenn die Isolations- und/oder Reflektionsschicht durchsichtig oder zumindest partiell durchsichtig ist. Hierfür kann beispielsweise ein engmaschiges Netz, eine gelochte Folie oder eine Folie mit durchsichtigen Fensterbereichen vorgesehen sein.

Eine einfache Ausführungsvariante, die sich vor allem für zylinderförmige Oxygenatoren eignet, sieht vor, dass der Oxygenator eine zentrale Öffnung mit einem dornförmigen Halteelement aufweist. Dann kann das dornförmige Halteelement auch das Heizelement und insbesondere die Strahlungsquelle aufweisen, um im Gehäuseraum fließendes Blut zu erwärmen.

Offenbart wird auch ein Verfahren zum Regeln der Wärmeabgabe an einem Heizelement eines Oxygenators, bei dem der Durchfluss des Blutes durch den Oxygenator und die Leistung einer der Durchfluss beeinflussenden Pumpe gemessen werden und in Abhängigkeit davon die Heizleistung eingestellt wird. Dabei kann der Receiver mehrere getrennt voneinander ansteuerbare Receiverteilelemente aufweisen, die so angesteuert werden, dass die Temperaturdifferenz zwischen der Temperatur des Blutes am Receiverteilelement und der Temperatur des Receiverteilelementes einen vorbestimmten Wert nicht übersteigt. Diese Verfahren eignen sich besonders für einen Oxygenator nach einem der vorhergehenden Ansprüche.

Ausführungsbeispiele erfindungsgemäßer Oxygenatoren sind in der Zeichnung dargestellt und werden im Folgenden näher beschrieben.

Es zeigt
Figur 1 einen bekannten Oxygenator mit Blut-, Gas-, und Wasserströmung,
Figur 2 schematisch einen mit infrarotem Licht bestrahlten Oxygenator,
Figur 3 eine Draufsicht auf den in Figur 2 gezeigten Oxygenator,
Figur 4 schematisch einen Oxygenator mit geschichteten Membranfasermatten und halbdurchlässig bedruckter Receiveroberfläche,
Figur 5 schematisch einen Oxygenator mit geschichteten Matten und getrennt ansteuerbaren Receiverteilelementen,
Figur 6 schematisch das Zusammenwirken von Komponenten für eine Algorithmus,
Figur 7 schematisch die mittleren Temperaturen über der Zeit,
Figur 8 schematisch die Temperatur über der Zeit an einem ersten Ort und
Figur 9 schematisch die Temperatur über der Zeit an einem zweiten Ort.

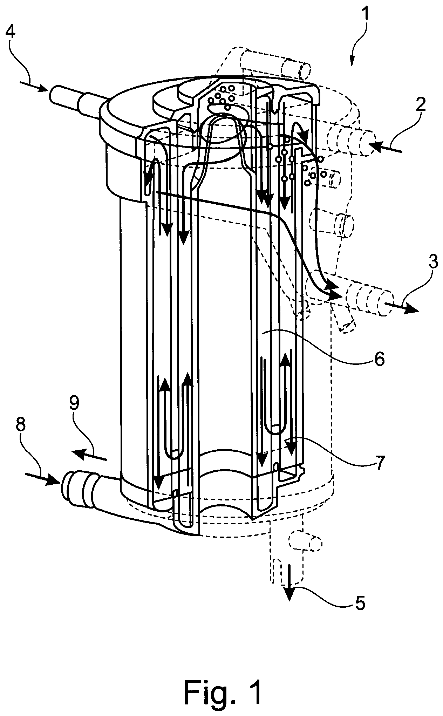

Der in Figur 1 gezeigte Oxygenator 1 hat einen Bluteinlass 2 und einen Blutauslass 3. Zur Gasversorgung sind ein Gaseinlass 4 und ein Gasauslass 5 vorgesehen. Im Wärmetauscher sind im radial inneren Bereich wasserdurchströmte Hohlfasern und im radial äußeren Bereich gasdurchströmte semipermeable Hohlfasern vorgesehen. Dadurch erfolgt radial innen eine Erwärmung durch am Wassereinlass 8 eintretendes und am Wasserauslass 9 austretendes Wasser, während im radial äußeren Bereich 7 ein Gasaustausch stattfindet. Zur genaueren Erläuterung eines derartigen Oxygenators wird auf die EP 765 683 B1 verwiesen.

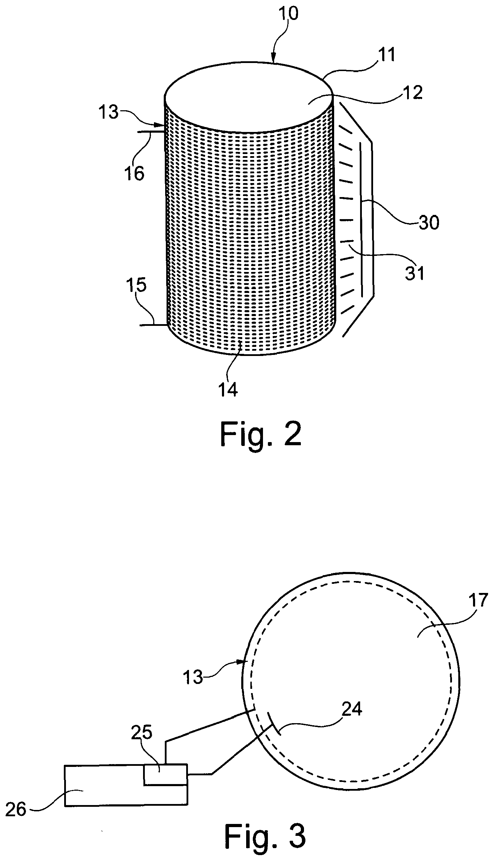

Bei dem in Figur 2 schematisch gezeigten Oxygenator bleibt der prinzipielle Aufbau im Wesentlichen erhalten und es wird auf den Wassereinlass 8, den Wasserauslass 9 und die wasserdurchströmten Hohlfasen verzichtet. Der Oxygenator 10 hat eine Gehäusewandung 11, die einen Gehäuseraum 12 umgibt. Dieser Gehäuseraum 12 hat, wie in Figur 1 gezeigt, einen Bluteinlass 2 und einen Blutauslass 3 und einen Gaseinlass 4 und einen Gasauslass 5. Als Receiver 13 dient eine in der Gehäusewandung 11 angeordnete halbdurchlässig mattschwarz bedruckte Fläche 14, die als Folie gleichmäßig um den Gehäuseraum 12 gewickelt ist. Die Folie 14 bildet einen Receiver, der warm wird, wenn am elektrischen Anschluss 15, 16 eine Spannung angelegt wird, damit die Strahlungsquelle 30 Infrarotlicht 31 aussendet.

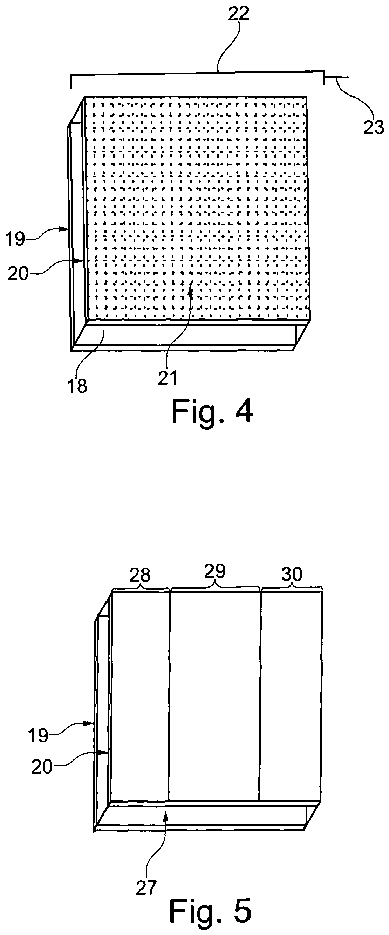

Der Oxygenator kann, wie in den Ausführungsbeispielen der Figuren 1 bis 3, aufgewickelte Hohlfasermatten 17 aufweisen, die im Gehäuseraum 12 angeordnet sind, oder, wie in den Figuren 4 und 5 gezeigt, gestapelte Matten 18 aus Hohlfasermembranen, die zwischen zwei Platten 19 und 20 angeordnet sind. Im Ausführungsbeispiel der Figur 4 ist auf der oberen Platte 20 eine halbdurchlässig mattschwarz bedruckte Fläche 21 vorgesehen. Eine Strahlungsquelle 22 ist mit einem elektrischen Anschluss verbunden, um eine Infrarotstrahlung auf die bedruckte Fläche 21 zu richten.

Die Figur 5 zeigt Hohlfasermatten 27 zwischen zwei Platten 19 und 20, von denen die obere Platte 20 eine halbdurchlässig mattschwarz bedruckte Fläche 21 aufweist. Schematisch sind drei Receiverteilelemente 28, 29, 30 angedeutet, die unterschiedlich strahlungsabsorptionsfähig sein können oder unterschiedlich bestrahlt werden können, um an verschiedenen Orten des Oxygenators unterschiedlich viel Wärme zu erzeugen. Die Receiverteilelemente 28, 29 und 30 können insbesondere so angesteuert werden, dass ein bestimmter Temperaturunterschied zwischen Receiverteilelement und Bluttemperatur am Receiverteilelement nicht überschritten wird.

Die Figur 3 zeigt schematisch einen Temperatursensor 24, der mit einer Temperaturregeleinrichtung 25 in Verbindung steht, die in einer Konsole 26 aufgenommen ist.

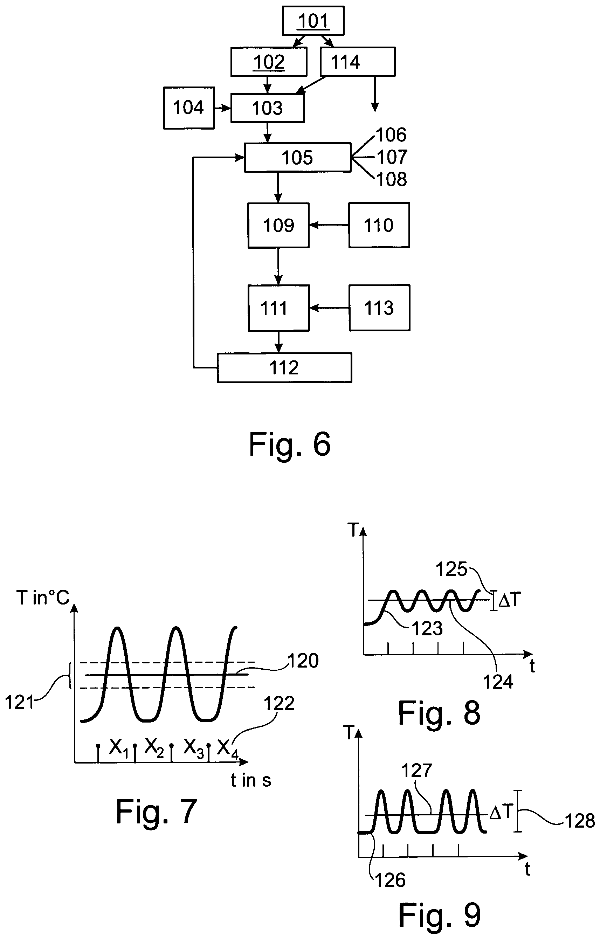

In den Figuren 6 bis 9 ist die algorithmische Steuerung nach Zeitintervallen in einer optimierten Form dargestellt. Aus einem realen Gasaustauscher wird ein Modell ermittelt. In diesem Gasaustauschermodell, das in der Figur 6 als Oxygenatormodell 114 zur Messpunktlokalisation eingezeichnet ist, werden Messpunkte definiert und es werden die Materialeigenschaften ermittelt, die als Parameter für die Berechnungen benötigt werden. An allen Messpunkten kann die Temperatur abgenommen werden.

In die Konsole werden durch die in einem ECMO-System bereits vorhandenen Sensoren, Blut- und Gasparameter eingespeist (KD). Anschließend wird eine Wunschtemperatur mit einer gemessenen Temperatur unter Berücksichtigung von Konsolenwerten (KD) verglichen. Dies wird individuell für jeden Messpunkt und jedes Heizelement vorgenommen. Dadurch ergeben sich verschiedene Toleranzen zwischen Messwert und Wunschtemperatur. Anschließend wird aus allen Parametern für jedes Heizelement die passende Heizfrequenz mit Heizhäufigkeit und Heizintensität gewählt, um möglichst blutschonend die Wunschtemperatur zu erreichen und anschließend zu halten. Diese Frequenzen können in einer Tabelle abgespeichert werden, um die Steuerung des Oxygenators später zu erleichtern.

In dem in Figur 6 dargestellten Algorithmus legt der Benutzer 101 die Wunschtemperatur 102 fest, die in die Konsole 103 eingegeben wird. Darüber hinaus werden die Blutfluss-, Gasfluss- und Druckparameter 104 in die Konsole eingegeben. Die Konsole veranlasst eine Temperaturmessung 105 um mittlere Temperaturen 106, 107 und 108 an unterschiedlichen Messpunkten des Oxygenators zu ermitteln. Der Vergleich 109 der Wunschtemperatur (WT) und der mittleren gemessenen Temperatur an unterschiedlichen Orten führt zum Differenzwert (AG). Dieser Wert wird mit den festgelegten Temperaturabweichungen 110 an unterschiedlichen Orten und den Konsolendaten (KD) wie beispielsweise dem Blutfluss verrechnet. Daraus ergibt sich die Grundlage für eine individuelle Regelung 111 der Heizelemente auf der Basis des Abgleiches (AG) der Temperaturabweichungen (AT) und den Konsolendaten (KD). Mit diesen Werten können die Heizelemente 112 angesteuert werden. Außerdem kann die individuelle Regelung noch vom Heizalgorithmus 113 beeinflusst werden, der aus einer Tabelle ausgewählt wird und sich aus den gemessenen Parametern ergibt. Außerdem kann jede Änderung des Receivers (Farbe, Stellung, Schaltung etc.) zusätzlich zum Heizalgorithmus oder an Stelle des Heizalgorithmus zur Beeinflussung der Steuerung genutzt werden.

Die Wärmeabgabe der Heizelemente 112 wirkt auf die mit der Temperaturmessung gemessenen Temperaturen 105, wodurch eine Rückkopplung auf die gemessenen Temperaturen entsteht.

Hierzu zeigt die Figur 7 die Wunschtemperatur 120 in einem Koordinatensystem mit der Temperatur in °C über der Zeit in Sekunden. Der Lamda-Wert deutet die Wärmeleitfähigkeit an, die durch die Materialkonstanten beeinflusst ist und die dazu führt, dass Temperaturspitzen abgefangen werden. Mit x1, x2, x3 und x4 sind beispielhaft Temperaturmesspunkte 122 bezeichnet.

In den Figuren 8 und 9 ist für zwei Messorte der Temperaturverlauf über der Zeit dargestellt. Dabei wird der Temperaturverlauf 123 an einem ersten Messort in Figur 8 über der Zeit als Wellenlinie dargestellt, die um eine Temperatur 124 schwankt und ein Delta T (Δ T) 125 definiert. In entsprechender Weise ist in Figur 9 an einem zweiten Ort die aktuelle Temperatur 126 über einer mittleren Temperatur 127 aufgezeichnet, wodurch sich eine Temperaturabweichung Delta T (Δ T) 128 ergibt.

## Patentansprüche

1. Oxygenator (10) mit einer Gehäusewandung (11), die einen Gehäuseraum (12) mit einem Bluteinlass (2), einem Blutauslass (3), einem Gaseinlass (4) und einem Gasauslass (5) begrenzt, und einem Heizelement, um durch den Gehäuseraum (12) fließendes Blut zu temperieren, wobeidas Heizelement eine Strahlungsquelle und einen Receiver (13) aufweist, der die Strahlung der Strahlungsquelle in Wärme umwandelt, ***dadurch gekennzeichnet, dass*** der Receiver (13) an unterschiedlichen Orten im Gehäuseraum (12) eine unterschiedliche Heizleistung abgibt oder mehrere an verschiedenen Orten des Oxygenators positionierbare Receiverteilelemente (28, 29, 30) aufweist und dass der Receiver (27) mehrere getrennt voneinander ansteuerbare Receiverteilelemente (28, 29, 30) aufweist.

2. Oxygenator nach Anspruch 1, ***dadurch gekennzeichnet, dass*** die Strahlungsquelle (30) Infrarotlicht (31) abgibt und der Receiver (13) eine dunkle Fläche (14) aufweist.

3. Oxygenator nach Anspruch 2, ***dadurch gekennzeichnet, dass*** die Wellenlänge der Strahlungsquelle (30) einstellbar ist.

4. Oxygenator nach Anspruch 2 oder 3, ***dadurch gekennzeichnet, dass*** die Absorptionsfähigkeit des Receivers (13) geregelt veränderbar ist.

5. Oxygenator nach einem der vorhergehenden Ansprüche, ***dadurch gekennzeichnet, dass*** die Strahlungsquelle (30) geregelt ausrichtbar ist.

6. Oxygenator nach einem der vorhergehenden Ansprüche, ***dadurch gekennzeichnet, dass*** die Strahlungsquelle (30) einen Lüfter aufweist.

7. Oxygenator nach einem der vorhergehenden Ansprüche, ***dadurch gekennzeichnet, dass*** die Strahlungsquelle (30) eine vom Gehäuseraum (12) trennbare Einrichtung ist.

8. Oxygenator nach einem der vorhergehenden Ansprüche, ***dadurch gekennzeichnet, dass*** die Strahlungsquelle (30) an einem Gehäusehalter angeordnet ist.

9. Oxygenator nach einem der vorhergehenden Ansprüche, ***dadurch gekennzeichnet, dass*** die Strahlungsquelle (30) konzentrisch zum Gehäuseraum (12) angeordnet ist.

10. Oxygenator nach einem der vorhergehenden Ansprüche, ***dadurch gekennzeichnet, dass*** der Receiver (13) in der Gehäusewandung (11) angeordnet ist.

11. Oxygenator nach einem der vorhergehenden Ansprüche, ***dadurch gekennzeichnet, dass*** der Receiver (13) im Gehäuseraum (12) angeordnet ist.

12. Oxygenator nach einem der vorhergehenden Ansprüche, ***dadurch gekennzeichnet, dass*** der Oxygenator Membranen wie insbesondere Hohlfasern (17) aufweist.

13. Oxygenator nach einem der vorhergehenden Ansprüche, ***dadurch gekennzeichnet, dass*** er mindestens einen Temperatursensor (24) aufweist.

14. Oxygenator nach einem der vorhergehenden Ansprüche, ***dadurch gekennzeichnet, dass*** er eine Temperaturregeleinrichtung (25) aufweist.

15. Oxygenator nach Anspruch 14, ***dadurch gekennzeichnet, dass*** die Temperaturregeleinrichtung (25) in einer Konsole (26) angeordnet ist.

16. Oxygenator nach Anspruch 14 oder 15, ***dadurch gekennzeichnet, dass*** die Temperaturregelung (25) die Temperatur des Receivers (13) an verschiedenen Orten individuell einstellt oder regelt.

17. Oxygenator nach einem der vorhergehenden Ansprüche, ***dadurch gekennzeichnet, dass*** er eine automatische Abschalteinrichtung für eine Überhitzung aufweist.

18. Oxygenator nach einem der vorhergehenden Ansprüche, ***dadurch gekennzeichnet, dass*** der Receiver (13) im Oxygenator zwischen Gaseinlass (4) und Gasauslass (5) angeordnet ist, um auch durch den Gehäuseraum (12) fließendes Gas zu temperieren.

19. Oxygenator nach einem der vorhergehenden Ansprüche, ***dadurch gekennzeichnet, dass*** der Receiver eine Wärmeleiteinrichtung für eine Wärmeverteilung vom Receiver (13) im Gehäuseraum (12) aufweist.

20. Oxygenator nach einem der vorhergehenden Ansprüche, ***dadurch gekennzeichnet, dass*** er eine Isolationsschicht aufweist, um im Gehäuseraum fließendes Blut zu isolieren.

21. Oxygenator nach einem der vorhergehenden Ansprüche, ***dadurch gekennzeichnet, dass*** er eine Reflexionsschicht aufweist, um Wärmestrahlung von im Gehäuseraum fließendem Blut zu reflektieren.

22. Oxygenator nach Anspruch 20 oder 21, ***dadurch gekennzeichnet, dass*** die Schicht durchsichtig oder partiell durchsichtig ist.

## Claims

1. Oxygenator (10) with a housing wall (11), which delimits a housing space (12), with a blood inlet (2), a blood outlet (3), a gas inlet (4) and a gas outlet (5), and a heating element to control the temperature of the blood flowing through the housing space (12), wherein the heating element has a radiation source and a receiver (13), which converts the radiation of the radiation source into heat, **characterized in that** the receiver (13) delivers a different heating power at various locations in the housing space (12), or has a plurality of receiver components (28, 29, 30), which can be positioned at various locations in the oxygenator, and **in that** the receiver (27) has a plurality of receiver components (28, 29, 30), which can be controlled separately from one another.

2. Oxygenator according to claim 1, **characterized in that** the radiation source (30) emits infrared light (31) and the receiver (13) has a dark surface (14).

3. Oxygenator according to claim 2, **characterized in that** the wavelength of the radiation source (30) is adjustable.

4. Oxygenator according to claim 2 or 3, **characterized in that** the absorption capacity of the receiver (13) is can be altered in a regulated manner.

5. Oxygenator according to any one of the preceding claims, **characterized in that** the radiation source (30) can be aligned in a regulated manner.

6. Oxygenator according to any one of the preceding claims, **characterized in that** the radiation source (30) has a fan.

7. Oxygenator according to any one of the preceding claims, **characterized in that** the radiation source (30) is a device that can be set apart from the housing space (12).

8. Oxygenator according to any one of the preceding claims, **characterized in that** the radiation source (30) is arranged on a housing holder.

9. Oxygenator according to any one of the preceding claims, **characterized in that** the radiation source (30) is arranged concentrically with the housing space (12).

10. Oxygenator according to any one of the preceding claims, **characterized in that** the receiver (13) is arranged in the housing wall (11).

11. Oxygenator according to any one of the preceding claims, **characterized in that** the receiver (13) is arranged in the housing space (12).

12. Oxygenator according to any one of the preceding claims, **characterized in that** the oxygenator has membranes such as, in particular, hollow fibers (17).

13. Oxygenator according to any one of the preceding claims, **characterized in that** it has at least one temperature sensor (24).

14. Oxygenator according to any one of the preceding claims, **characterized in that** it comprises a temperature regulation device (25).

15. Oxygenator according to claim 14, **characterized in that** the temperature regulation device (25) is arranged in a console (26).

16. Oxygenator according to claim 14 or 15, **characterized in that** the temperature regulator (25) individually adjusts or regulates the temperature of the receiver (13) at various locations.

17. Oxygenator according to any one of the preceding claims, **characterized in that** it has an automatic cut-off device for use in the event of overheating.

18. Oxygenator according to any one of the preceding claims, **characterized in that** the receiver (13) is arranged in the oxygenator between the gas inlet (4) and the gas outlet (5), so as to control also the temperature of gas flowing through the housing space (12).

19. Oxygenator according to any one of the preceding claims, **characterized in that** the receiver has a heat conduction device for the distribution of heat from the receiver (13) in the housing space (12).

20. Oxygenator according to any one of the preceding claims, **characterized in that** it has an insulating layer to insulate the blood flowing in the housing space.

21. Oxygenator according to any one of the preceding claims, **characterized in that** it has a reflective layer so as to reflect heat radiation from the blood flowing in the housing space.

22. Oxygenator according to claim 20 or 21, **characterized in that** the layer is transparent or partially transparent.

## Revendications

1. Oxygénateur (10) muni d'une paroi de boîtier (11) qui délimite un espace de boîtier (12) avec une entrée de sang (2), une sortie de sang (3), une entrée de gaz (4) et une sortie de gaz (5), et un élément chauffant afin de tempérer du sang circulant à travers l'espace de boîtier (12), dans lequel l'élément chauffant présente une source de rayonnement et un récepteur (13) qui convertit le rayonnement de la source de rayonnement en chaleur, ***caractérisé en ce que*** le récepteur (13) émet une puissance de chauffage différente à différents emplacements dans l'espace de boîtier (12) ou présente une pluralité de sous-éléments de récepteur (28, 29, 30) qui peuvent être positionnés à différents emplacements de l'oxygénateur, et **en ce que** le récepteur (27) comporte une pluralité de sous-éléments de récepteur (28, 29, 30) qui peuvent être commandés séparément les uns des autres.

2. Oxygénateur selon la revendication 1, ***caractérisé en ce que*** la source de rayonnement (30) émet une lumière infrarouge (31) et le récepteur (13) présente une surface sombre (14).

3. Oxygénateur selon la revendication 2, ***caractérisé en ce que*** la longueur d'onde de la source de rayonnement (30) est réglable.

4. Oxygénateur selon la revendication 2 ou 3, ***caractérisé en ce que*** la capacité d'absorption du récepteur (13) peut être modifiée de manière régulée.

5. Oxygénateur selon l'une quelconque des revendications précédentes, ***caractérisé en ce que*** la source de rayonnement (30) peut être orientée de manière régulée.

6. Oxygénateur selon l'une quelconque des revendications précédentes, ***caractérisé en ce que*** la source de rayonnement (30) présente une soufflante.

7. Oxygénateur selon l'une quelconque des revendications précédentes, ***caractérisé en ce que*** la source de rayonnement (30) est un dispositif pouvant être séparé de l'espace de boîtier (12).

8. Oxygénateur selon l'une quelconque des revendications précédentes, ***caractérisé en ce que*** la source de rayonnement (30) est agencée sur un support de boîtier.

9. Oxygénateur selon l'une quelconque des revendications précédentes, ***caractérisé en ce que*** la source de rayonnement (30) est agencée de manière concentrique par rapport à l'espace de boîtier (12).

10. Oxygénateur selon l'une quelconque des revendications précédentes, ***caractérisé en ce que*** le récepteur (13) est agencé dans la paroi de boîtier (11).

11. Oxygénateur selon l'une quelconque des revendications précédentes, ***caractérisé en ce que*** le récepteur (13) est agencé dans l'espace de boîtier (12).

12. Dispositif selon l'une quelconque des revendications précédentes, ***caractérisé en ce que*** l'oxygénateur présente des membranes, comme notamment des fibres creuses (17).

13. Oxygénateur selon l'une quelconque des revendications précédentes, ***caractérisé en ce qu***'il présente au moins un capteur de température (24).

14. Oxygénateur selon l'une quelconque des revendications précédentes, ***caractérisé en ce qu***'il présente un dispositif de régulation de température (25).

15. Oxygénateur selon la revendication 14, ***caractérisé en ce que*** le dispositif de régulation de température (25) est agencé dans une console (26).

16. Oxygénateur selon la revendication 14 ou 15, ***caractérisé en ce que*** le dispositif de régulation de température (25) ajuste ou régule individuellement la température du récepteur (13) à différents emplacements.

17. Oxygénateur selon l'une quelconque des revendications précédentes, ***caractérisé en ce qu***'il comprend un dispositif d'arrêt automatique en cas de surchauffe.

18. Oxygénateur selon l'une quelconque des revendications précédentes, ***caractérisé en ce que*** le récepteur (13) est agencé dans l'oxygénateur entre l'entrée de gaz (4) et la sortie de gaz (5) afin également de tempérer du gaz circulant à travers l'espace de récepteur (12).

19. Oxygénateur selon l'une des revendications précédentes, ***caractérisé en ce que*** le récepteur présente un dispositif de conduction de chaleur pour une distribution de la chaleur depuis le récepteur (13) jusque dans l'espace du boîtier (12).

20. Oxygénateur selon l'une quelconque des revendications précédentes, ***caractérisé en ce qu'***il présente une couche isolante permettant d'isoler le sang circulant dans l'espace de récepteur.

21. Oxygénateur selon l'une quelconque des revendications précédentes, ***caractérisé en ce qu***'il comprend une couche réfléchissante permettant de réfléchir le rayonnement thermique du sang circulant dans l'espace de récepteur.

22. Oxygénateur selon la revendication 20 ou 21, ***caractérisé en ce que*** la couche est transparente ou partiellement transparente.
